(19) Europäisches Patentamt
European Patent Office
Office européen des brevets

(11) EP 0 705 603 B1

(12) EUROPEAN PATENT SPECIFICATION

(45) Date of publication and mention of the grant of the patent:
**16.08.2000 Bulletin 2000/33**

(51) Int Cl.⁷: **A61K 31/195**
// (A61K31/195, 31:095)

(21) Application number: **95306587.7**

(22) Date of filing: **19.09.1995**

(54) **Use of non-toxic cysteine sulfoxide derivatives in the treatment of cancer**

Verwendung von nicht toxischen Cystein-Sulfoxid-Derivaten für die Behandlung von Krebs

Utilisation de dérivés non-toxiques de cystéin sulfoxide pour le traitement du cancer

(84) Designated Contracting States:
**AT BE CH DE DK ES FR GB GR IE IT LI LU MC NL PT SE**
Designated Extension States:
**LT LV SI**

(30) Priority: **20.09.1994 GB 9419061**

(43) Date of publication of application:
**10.04.1996 Bulletin 1996/15**

(73) Proprietor: **Holt, John Alfred Gorton**
**Perth, Western Australia 6009 (AU)**

(72) Inventor: **Holt, John Alfred Gorton**
**Perth, Western Australia 6009 (AU)**

(74) Representative: **Brown, David Leslie et al**
**Page Hargrave**
**Southgate**
**Whitefriars**
**Lewins Mead**
**Bristol BS1 2NT (GB)**

(56) References cited:
**EP-A- 0 531 031** **EP-A- 0 616 803**
**US-A- 3 892 852**

- **FOOD AND CHEMICAL TOXICOLOGY, vol. 31, no. 7, 1993 GREAT BRITAIN, pages 491-495, H. S. MARKS ET AL 'EFFECTS OF S-METHYL CYSTEINE SULPHOXIDE AND ITS METABOLITE METHYL METHANE THIOSULPHINATE, BOTH OCCURRING NATURALLY IN BRASSICA VEGETABLES, ON MOUSE GENOTOXICITY'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 705 603 B1

## Description

The present invention relates to therapeutic uses and compositions for use therein.

European Patent Application No. 0531031 describes a cancer therapy, and compositions for use therein, in which an effective amount of a non-toxic organic disulfide is administered to a patient and while said disulfide is present at a cancer site of the patient there is administered to the cancer site an effective dose of microwave electromagnetic radiation of frequency in the range of about 400-450 MHz.

European Patent Application No. 0616803 describes the use of such methods in enhancing blood T-cell count in immunodeficient (e.g. HIV-positive) individuals.

In both documents, cystine, which has the formula

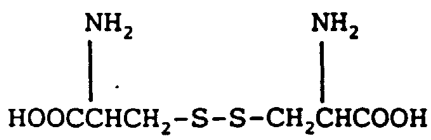

(I)

and non-toxic salts thereof are stated to be preferred disulfide agents.

Oxidised glutathione

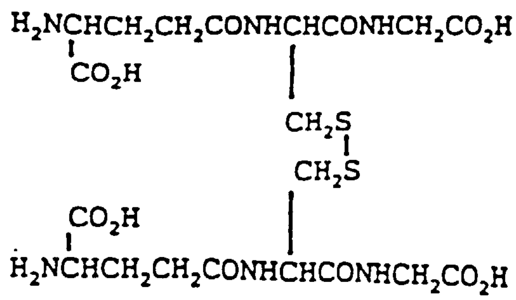

(II)

and non-toxic salts thereof are stated in EP-A-0616803 to be alternative preferred disulfide agents in the context of enhancement of a patient's T-cell count.

T-butyl hydroperoxide, which has the formula

$$CH_3C(CH_3)_2OOH \quad \text{(III)}$$

is stated in EP-A-0531031 to be desirably used as an oxidising agent to prevent degradation of the disulfide agents.

The present invention is based on my surprising finding that the above methods may be performed at least as well by using certain selected S-substituted cysteine sulfoxides in place of, or in addition to, the previously described active agents, administration of the sulfoxides being desirably preceded by administration of t-butyl hydroperoxide as oxidising agent.

These sulfoxide compounds are believed to prevent the exponential production of electrons in the redox cycle involving oxidised glutathione (II) and glutathione, which has the formula:

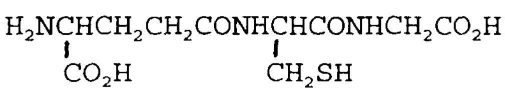

(IV).

In the transformation of two molecules of glutathione to one molecule of oxidised glutathione two electrons are produced. Only one electron is required to convert one molecule of oxidised glutathione to two molecules of glutathione. This redox cycle is therefore believed to be a producer of energy in exponential proportion to time. As cancer

grows uniquely by exponential increments this reaction is believed to be its source of energy.

The invention may therefore be stated to provide the use of a non-toxic S-substituted cysteine sulfoxide of the general formula

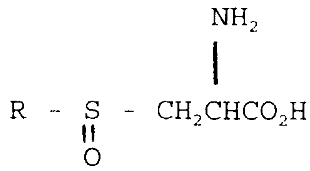

(V)

or a non-toxic salt thereof, in which R is a $C_{1-4}$ alkyl or a $C_{2-4}$ alkenyl group, for the manufacture of a composition or compositions for use as a medicament in a method of cancer therapy in a patient in which there is intravenously administered to a cancer site of the patient an effective amount of said non-toxic cysteine sulfoxide or salt thereof, and while said non-toxic cysteine sulfoxide is present at the cancer site of the patient there is administered to the cancer site an effective dose of microwave electromagnetic radiation of frequency in the range of 425-450 MHz.

The expression "non-toxic" used herein refers to an acceptably low level of toxicity when a compound is present at an effective amount, and not necessarily to a complete absence of toxic effects. In particular, compounds which have only a temporary toxic effect and do no significant permanent harm, or for which any toxic effects can readily be counteracted by the administration of a non-toxic "antidote" agent, will be referred to and understood as "non-toxic" herein.

The expression "cancer" used herein refers to all forms of neoplasia and precursors thereof. There may particularly be mentioned a malignant neoplasm of the epithelium (for example, a carcinoma such as squamous cell carcinoma, basal cell carcinoma, transitional cell carcinoma, adenocarcinoma or cystadenocarcinoma), pigment cells (for example, a malignant melanoma), neural and brain tissue (for example, neurofibrosarcoma, malignant glioma, medulloblastoma or neuroblastoma), kidney tissue (for example, a nephroblastoma), eye tissue (for example, a retinoblastoma), connective tissue (for example, a sarcoma or meningioma such as fibrosarcoma, liposarcoma, Kaposi's sarcoma, chondrosarcoma, osteosarcoma, myosarcoma, leiomyosarcoma, rhabdomyosarcoma, angiosarcoma, haemangiosarcoma, lymphangiosarcoma or malignant meningioma), lymphoreticular and haematopoietic tissue (for example, a malignant non-Hodgkin's lymphoma, lymphocytic leukaemia, plasmacytoma and multiple myeloma, reticulum cell sarcoma, Hodgkin's disease, granulocytic leukaemia or monocytic leukaemia) or embryonic-originating tissue (for example, a malignant teratoma, teratocarcinoma or chorioncarcinoma). The method of the present invention may also be used to treat benign neoplasia.

The expression "cancer site" is not intended to be restricted to localised sites within a patient's body, but covers widespread and multiple sites such as occur, for example, in leukaemias or in advanced cancerous invasions.

If desired, more than one compound of formula V or non-toxic salt thereof may be used simultaneously or sequentially according to the invention. An oxidising agent, preferably t-butyl hydroperoxide (III), is desirably administered separately before the compound(s) of formula V or non-toxic salt(s) thereof, to prevent or restrict degradation of the active agent(s). Furthermore, there may also be used additional active agents selected from cystine (I), non-toxic salts thereof, oxidised glutathione (II) and non-toxic salts thereof. Mixtures of such compounds may also be used.

Without wishing to be bound by theory, it is believed that the normal body components cystine and oxidised glutathione will direct the electrons produced in the glutathione redox cycle from their normal function of powering mitosis, and that the compounds of formula V are non-toxic precursors of the glutathione redox cycle which will be synthesised into glutathione.

The compounds of formula V and their non-toxic salts, as well as cystine, oxidised glutathione and their non-toxic salts, are non-toxic in doses exceeding 100 mg per kilogram body weight.

In the compounds of formula V, R is optionally substituted and is preferably methyl, ethyl, propyl, butyl or allyl. An optically active form of the compound, or alternatively a racemic mixture, may be used. The laevorotatory (L) form is preferred. The non-toxic salts are preferably non-toxic acid or base addition salts, for example addition salts of strong acids or bases.

Specifically, the most preferred active agents of formula V are S-methyl-L-cysteine sulfoxide, S-ethyl-L-cysteine sulfoxide, S-propyl-L-cysteine sulfoxide, S-butyl-L-cysteine sulfoxide and mixtures thereof. A mixture of the methyl, ethyl and butyl sulfoxides (e.g. in a 3:2:1 weight ratio) has been found to be the particularly effective.

In the optimum *in vivo* method the sulfoxide active agents are used in association with the oxidising agent t-butyl hydroperoxide (III), to prevent reduction of the sulfoxides *in vivo*, particularly in the liver. A stock solution of 70% t-butyl hydroperoxide in water, diluted up to 60-fold (by volume) in normal (0.9%) saline, e.g. around 20-fold, may conveniently be used. It is preferred that the oxidising agent be intravenously administered in the *in vivo* methods, prior

to and separately from the sulfoxide active agents. It has been found that 60 ml of the above saline solution of oxidising agent can safely and effectively be administered over a period of about 5 minutes. The amount, formulation and intravenous administration route for the oxidising agent can readily be selected by a worker of ordinary skill in this art.

Cystine (and non-toxic salts thereof) and oxidised glutathione (and non-toxic salts thereof) may suitably be used in association with one or more oxidising agent, for example hydrogen peroxide, to prevent degradation of the disulfides to corresponding thiols at least prior to administration to the patient. One ml of 10% hydrogen peroxide aqueous solution per gram of the disulfide to be administered is typically adequate, although the amount may be varied according to the particular requirement of the active agents used, in a manner which will be well within the capacity of a worker of ordinary skill in this art.

The composition form(s) by which the active agent(s) and any oxidising agent(s) is or are administered to the patient may take any suitable form and employ any suitable conventional pharmaceutical carrier(s) or excipient(s). The composition form is preferably an aqueous solution or suspension suitable for intravenous drip, infusion or injection. Intravenous drip, infusion or injection should be used as the administration route by virtue of its rapid delivery of the active agent(s) to the bloodstream or to a cancer site with minimal degradation of the active agent(s).

The solution or other composition form may if desired include additional components such as salts (e.g. sodium chloride), solubilising and/or suspending agents and the like. As mentioned above, oxidants such as hydrogen peroxide or organic hydroperoxides may be used.

There may particularly be mentioned a composition for use in a method of therapy according to the invention, comprising an aqueous solution of a non-toxic cysteine sulfoxide of general formula V, e.g. methyl, ethyl, propyl or butyl cysteine sulfoxide (or a non-toxic salt thereof) at a concentration of up to approximately 100 g/l (e.g. approximately 90-110 g/l), together with cystine and/or oxidised glutathione and/or a non-toxic salt thereof at a concentration of up to approximately 40 g/l (e.g. approximately 30-50 g/l).

There may also particularly be mentioned a kit of two or more compositions containing active agents, selected from compounds of formulae I, II, III and V and non-toxic salts thereof, for rapidly sequential administration in a method of therapy according to the invention, a first composition comprising an aqueous solution containing a first active agent selected from the agents as defined above, and a second composition comprising an aqueous solution containing a second active agent selected from the agents as defined above, with the provisos that at least one of said first and second active agents is a compound of general formula V or a salt thereof and that the composition to be administered first includes the compound of formula III; the said first and second compositions being optionally together with further composition(s) and instructional literature for the therapy.

The compositions are prepared by standard mixing techniques that will be readily apparent to those skilled in this art. In the case of sparingly soluble active agents a suitable solubilising agent should first be dissolved in the aqueous medium.

Aqueous solutions of the active agent(s) of formula V and their salts at concentrations of up to around 100 grams per litre are convenient, enabling a daily injection volume of 50-60 ml to deliver around 5 grams of the desired sulfoxide intravenously. In the case of a mixture of active agents of formula V, a total weight of up to around 15 grams (e.g. 12 grams) in a volume of around 60 ml is convenient.

If cystine is present, an aqueous solution of L-cystine at a concentration of approximately 40 grams per litre is convenient, enabling a daily injection volume about 50 to 60 ml, delivering a daily dose of up to approximately 2 g. To assist the passing of L-cystine into solution, a solubilising agent such as N-methyl-D-glucamine is also conveniently present in the solution. A small amount of an oxidant such as hydrogen peroxide may also be present to prevent the L-cystine from degrading to its thiol cysteine prior to administration. To avoid any risk of nausea, L-cystine should be injected slowly.

If oxidised glutathione is present, an aqueous solution of oxidised glutathione at a concentration of approximately 40 grams per litre is convenient, enabling a daily injection volume of about 50 to 60 ml, to deliver approximately 2 g of oxidised glutathione.

After administration, to the patient, of the active agent(s) according to the invention and any required oxidising agent(s), the microwave radiation should normally be begun to be administered between about 1 and 5 minutes later.

To administer *in vivo* the microwave radiation, which covers in the case of cancer therapy at least the cancer site(s) or optionally the entire body of the patient if that is most convenient, or in the case of T-cell enhancement preferably the entire body, the patient is brought close to a suitable number of microwave antennae (e.g. 3 or 4 antennae arranged to encircle the patient's body) and the antennae energised to transmit UHF microwave radiation. Normally in the case of cancer therapy an area of at least about 30 cm radius around each cancer site should be irradiated, and for this purpose the patient may conveniently be moved under the antennae.

The total antennae power should preferably be less than about 4 kW (e.g. adjustable between about 1 and about 2 kW) in order to remain tolerable to the average patient. Patients may preferably be treated in two or more sessions, preferably three sessions, per day at daily intervals or every other or every third day for a treatment period of up to about four weeks.

Preferably, each session involves administration of the oxidising and active agent(s) followed over the subsequent 30 minutes by from 1 to about 5 exposures (e.g. three exposures) to the microwave radiation, each exposure to the radiation lasting for about 5 to 10 minutes, with a 2 to 5 minute rest period between exposures. The total length of treatment on each session day will be dependent on the length of time over which the agent(s) remain at the cancer site or in the bloodstream without substantial loss, and the length of rests between exposures which the patient requires. For optimum results each session should be terminated within 30 minutes of the first agent being administered (by intravenous injection). Small bodies (e.g. children) and adults can normally tolerate the same radiation power of about 1.6 to 2 kW.

Therapy using injections of t-butyl hydroperoxide (III) followed by compound V and optionally cystine (I) and/or oxidised glutathione (II), followed 1 to 5 minutes later by suitable microwave radiation, produces immediate improvement, at optimum after approximately 5 to 25 days later.

As mentioned above, the microwave radiation should be in the frequency range of about 425-450 MHz, most preferably 432-436 MHz (e.g. about 434 MHz).

The antennae are suitably standard commercially available microwave antennae of a convenient size and shape to accommodate the shape of the patient's body. Each antenna may consist of a single circular turn approximately 19 cm in diameter (the diameter being chosen to resonate at the frequency of the transmitter), the plane of the circular antenna being held substantially tangential to the curvature of the patient's body, and within about 10 cm from the patient's skin. Such a circular antenna may preferably be provided with adjustable inductor coils in known manner to emit predominantly H-wave microwave radiation of wavelength between about 65 and 75 cm (most preferably about 69 cm) towards the patient's body. While the presence of E-wave radiation will not necessarily hinder the efficacy of the radiation, it can lead to greater heating of the patient's skin, which is undesirable. Folded dipole antennae may alternatively be used, although these are less preferred.

Each antenna is suitably connected to a UHF generator by a coaxial cable via circulators to prevent adverse interaction between antennae. Each generator may if desired be set at a slightly different frequency at or around a central frequency in the range already mentioned (preferably about 434 MHz). Each antenna is preferably energised at about 400 W.

All equipment within about 2 metres of the antennae should be of non-metallic materials such as wood or plastic, including for example the patient support table.

## EXAMPLE

### Compositions and administration volumes

Aqueous solutions of t-butyl hydroperoxide are prepared by dissolving 50 ml of a 70% aqueous solution of t-butyl hydroperoxide in 1 litre of normal (0.9%) saline. 15-30 ml of this solution is given intravenously over a period of up to about 15 minutes.

Aqueous solutions of methyl, ethyl, propyl and butyl cysteine sulfoxide are prepared by dissolving 100 gram of the respective sulfoxide in 1 litre of normal (0.9%) saline. 30-60 ml of this solution (delivering an approximate 2 to 6 gram dose of the sulfoxide) is given intravenously.

If used, an aqueous solution of L-cystine is prepared by first dissolving 80 grams of N-methyl-D-glucamine in 1 litre of normal (0.9%) saline and the dissolving in 40 g of L-cystine. 50 to 60 ml of the solution (delivering an approximately 2 g dose of cystine) is given intravenously.

If used, an aqueous solution of oxidised glutathione is prepared by dissolving 40 grams in 1 litre of normal (0.9%) saline. 50-60 ml of this solution (delivering an approximately 2 g dose of oxidised glutathione) is given intravenously.

### Microwave Therapy

The t-butyl hydroperoxide solution is administered by intravenous infusion over a period of up to about 15 minutes. This is then followed immediately by intravenous infusion of the sulfoxide, optionally with cystine and/or oxidised glutathione solutions, and then (within three minutes) Ultra High Frequency microwave radiation is commenced and is delivered over the next 20 - 30 minutes. 1 or 2 rest intervals of 2 to 3 minutes each are permitted during this application. Three such treatment sessions may be undertaken per day.

This can be repeated daily as often as is necessary to cause resolution of part or all of the cancer or enhancement of the patient's T-cell count.

**Toxicity**

No symptoms suggestive of any toxicity have been noted after the intravenous administration of t-butyl hydroperoxide, L-cystine, oxidised glutathione or methyl, ethyl, propyl, butyl or allyl cysteine sulfoxide. No immediate or late changes in blood pressure, renal or liver function, electrolytes or haematology have been seen from the method.

**Contra-indications**

Most previous cytotoxic chemotherapy, however long ago, which will have destroyed the insulating properties of normal tissue so that cancer and normal tissues will have become electrically indistinguishable. This will therefore constitute a contra-indication to the present method in cancer therapy.
A prime contraindication is the presence of large collections of fluid in the pleural or peritoneal cavities. Static collections of fluid which contain high concentrations of salts have heat generated in them by the application of these magnetic fields. Patients with pleural or peritoneal effusions have blockage of the lymphatics draining these areas and also involvement of the pleural and peritoneal membranes. Unless these cavities are dry (less than 100 ml fluid when treated) the heating effect aggravates the irritation of the pleural and peritoneal cavities and the effusions become worse. Recommendations for treatment of such patients are as follows:

A: Peritoneal effusions. A Le Veen shunt or equivalent should be inserted prior to treatment and must be working so that the peritoneal cavity is kept dry. This drains the ascitic fluid back into the circulation.

B: Pleural effusions. A similar shunt can be used but is much less satisfactory. Pleural paracentesis to drain the cavity completely together with introduction of some agent causing sclerosis of the pleural cavity which obliterates it is more effective. The introduction of talc, tetracycline or another sclerosing agent appears the best way of preventing failure in the treatment of the underlying cancer.

Another contraindication to this treatment is where cancer of the stomach or bowels is causing partial obstruction to the gut. Treatment can cause breakdown of some of the cancer with a perforation of the bowel above the obstruction. All patients with bowel cancer should be fully assessed by a surgeon and if there is any evidence of obstruction the primary cancer should be removed or exteriorised by colostomy or some other similar procedure. This avoids the likelihood of perforation which can be lethal.
Regarding the HIV infections, any stage of the disease even up to frank AIDS with or without secondary infections and/or cancers are treatable. It is rational, however, to suggest that treatment when the T4 cell count is very low or zero could not raise the T4 cell count. There may be no stem cells left in the marrow, spleen, etc. with which to re-populate the normal T4 cells in the blood even if some of the virus is removed.
The following Case Histories are presented to show, without limitation, clinical applications of the method of the invention.
The first case history is presented in full; the remainder as summaries in note form. MCO = methyl cysteine sulfoxide; ECO = ethyl cysteine sulfoxide; BCO = butyl cysteine sulfoxide; TBH = t-butyl hydroperoxide. Dosages and formulations are as described above.

**CASE HISTORY 1**

GT, an HIV-positive male then aged 31, was referred with a three month history of increasing nasal congestion, enlarged lymph nodes both sides of the neck and a bone marrow biopsy which revealed the marrow to be extensively infiltrated with malignant lymphoma cells. Biopsy of nasopharyngeal tumour and a node in the neck showed a high grade immunoblastic malignant lymphoma. He had previously been given a course of radiotherapy to the nasal and post-nasal regions which caused considerable reduction in the malignant mass in the nasopharynx. Cytotoxic chemotherapy was precluded because of his HIV infection. Before radiotherapy his T4 cell count was 170, CD8 count 800 absolute numbers. CD4/CD8 ratio 0.35. After radiotherapy his T4 absolute cell count was less than 100, his T8 cell count was 480. Treatment with the method of the invention over an 18 day period elevated his T4 count to 300. A second course was given over a 21 day period nearly three months later. Bone marrow investigation revealed complete disappearance of his lymphatic lymphoma. All clinical evidence of node enlargement both sides of the neck, axillae and groins had disappeared, nasopharynx was completely clear on examination. Peripheral white cell count 4,700 with a normal distribution, red cell count 4.9. haemoglobin 141 grams per litre. Platelets 225,000 per cubic millimetre. He was completely well without any symptoms referable to his acute blastic lymphoma of his HIV. A secondary thrush infection of his mouth and throat had disappeared.
By 21 months later his T4 cell count had reduced to 210 with a T8 count of 528. Ratio 2.70. A further course

of treatment according to the invention was then given over an 18 day period. Secondary thrush infection and low grade respiratory tract infection completely disappeared within eight weeks. His T4 cell has increased to 600. He is being monitored and will be treated in due course if necessary.

## CASE HISTORY 2

LB (a female aged about 52 at onset of disease): Diagnosis - Adenosquamous carcinoma floor of mouth. Biopsy of a lump under tongue to left of midline together with biopsy of node in the left upper cervical region. Both positive for adenosquamous carcinoma. Primary and secondaries in the neck were resected. Presented nine months later with lymph node (1.5 cm diameter) recurrence just above the greater cornu of the hyoid on the left together with a smaller nodule behind the angle of the jaw and a submandibular triangle node (each 1 cm diameter). Axillae clear, chest x-ray clear. 15 day course of MCO and TBH according to the invention immediately thereafter. Clinical examination eight months later. Clear of disease.

## CASE HISTORY 3

JB (a female aged about 38 at onset of disease): History - a mass developed in the lateral compartment of the left calf in the peroneal muscle. The compartment was opened and the mass was found to encroach on the common peroneal nerve. Nerve was preserved but incomplete tumour excision was carried out. Pathology is a myxoid liposarcoma 14 cm long from the origin of the peroneus brevis muscle to its tendon. Reviewed one month later with recurrence: swelling of the left calf, a healed surgical incision and thickening beneath it. Chest x-ray was clear. 15 day course of ECO and TBH according to the invention commenced two weeks later. Complete resolution of all the oedema of the leg and restoration to normal anatomy apart from the previous surgical muscle loss. CT and x-ray appearances within normal limits. Reviewed by orthopaedic specialist 2.5 years later and no evidence of recurrence.

## CASE HISTORY 4

JB (a male aged about 69 at onset of disease): History - lower third of oesophagus removed plus stomach removed for a gastric adenocarcinoma involving the fundus of the stomach and lower part of the oesophagus. Presented 2.5 years later with obstruction due to proven recurrence at the lower end of his oesophagus. Three treatment courses according to the invention given immediately thereafter: 10 days using ECO and MCO only. Then 19 days rest. Then 16 days using ECO and MCO with TBH. Dramatic improvement occurred and swallowing returned to normal. X-ray 2 months later showed some irregularity and a third course of treatment according to the invention was given as before. Complete resolution of tumour has occurred. All symptoms disappeared and he regained the lost weight.

## CASE HISTORY 5

DE (a male aged about 55 at onset of disease): Transitional cell cancer of the bladder at the mouth of his diverticulum. Partial cystectomy to remove diverticulum and a cancer and was well for three years. Several small malignancies limited to the mucosa or infiltrating the muscle were then discovered; followed and diathermied at intervals. Grade 3 rapidly growing transitional cell carcinoma recurrence was biopisied 8 years after said partial cystectomy. Multiple other lesions were present throughout the mucosa of the bladder. A course of 15 treatments according to the invention using MCO, ECO and TBH. Two years later his bladder was clear.

## CASE HISTORY 6

KS (a male aged about 66 at onset of disease): Diagnosis: secondary adenocarcinoma of the left frontal lobe of brain or a primary in the choroid plexus. Increasing memory loss, spatial disorientation, mood swings and irritability. Scan showed right frontoparietal lobe malignancy. Craniotomy and partial removal of the lesion found to be a papillary type adenocarcinoma most likely from the choroid plexus. Chest x-ray clear, CT scan of abdomen etc normal. Biopsy of the bed of the tumour revealed active malignancy before the craniotomy was closed. Referred for radiotherapy treatment and he had 2300 rads. Referred 6 weeks after craniotomy operation with further symptoms suggesting activity of his disease. A CT scan was suggestive of malignancy in the right anterior horn of the ventricle which was the site of the base of the tumour originally. 15 day treatment according to the invention using ECO, MCO and TBH. General condition continued to improve and he has had no further symptoms of active disease. CT scan one month after treatment - No conclusive evidence of residual malignancy.

**CASE HISTORY 7**

RG (a male aged about 51 at onset of disease): Heavy smoker. Presented with symptoms suggesting a stone in the right ureter but cystoscopy showed invasive transitional cell carcinoma of the bladder with blockage of the right ureteric orifice. Full course of chemotherapy followed by a full course of radiotherapy. 4 years later showed scarring, possible evidence of recurrence. 2.5 years thereafter intravenous pilogram showed a defect in the right side of the bladder with recurrence of blockage of the right ureter. Biopsy confirmed transitional cell carcinoma with invasion of muscle. The mass was visible 2 cm in diameter in the bladder on x-rays. Treated according to the invention with ECO, MCO and TBH over 28 days. X-ray of bladder showed complete disappearance of the tumour with restoration and normal bladder outline.

**CASE HISTORY 8**

ER (a female aged about 76 at onset of disease): Diagnosis: carcinoma of the rectum. Attempted removal was impossible because the tumour was extending upwards and stuck to the left side of the pelvis. Referred and clinical examination revealed a large mass of malignancy just to the lateral side of the colostomy in the left iliac fossa which had been performed to bypass the residual tumour. Treatment over 24 days according to the invention using BCO, ECO and TBH. CT scan performed 5 months after treatment and showed considerable resolution of the mass. Continued clinical improvement and patient was asymptomatic one year after treatment. Reviewed by the surgeon who said there was no clinical evidence of recurrence of her disease at that time.

**CASE HISTORY 9**

VS (a male aged about 70 at onset of disease): Minor constipation for one month and an abnormal mass in the left side of the pelvis proven to be an adenocarcinoma of the sigmoid colon. This was removed and end to end anastomosis performed but spread of the malignancy to the appendix, pelvic wall and lower abdominal cavity was present. Treated according to the invention over 18 days with MCO and TBH. Examined one month after treatment and his ultrasound and CT scan suggested he had developed a small metastasis in the liver. Second course of treatment according to the invention over 18 days using MCO, ECO, BCO and TBH. The shadows in his liver disappeared on the ultrasound scan when seen two months later. All investigations, liver function tests, etc within normal limits; clinically normal.

**CASE HISTORY 10**

AK (a male aged about 54 at onset of disease): Heavy smoker. A routine x-ray showed a mass in the right hilum and right lower lobe. Biopsy proved small cell carcinoma of the right lung. He declined chemotherapy and radiotherapy after opinions had told him that these were methods which would not cure him. A thoracotomy was performed and the primary was removed. However some residual disease remained and enlarged mediastinal nodes were present which were proven positive for cancer. Treatment according to the invention with MCO, ECO and TBH over 17 days, two months after thoracotomy. Reviewed by the surgeon 5 months after treatment. Clinical and x-ray investigations and all haematology within normal limits.

**CASE HISTORY 11**

AN (a male aged about 77 at onset of disease): Adenocarcinoma of the prostate diagnosed. He had a bilateral orchidectomy and as a result of the orchidectomy his PSA reading fell from 64 to 14. His PSA slowly rose to 16 and he had incomplete control with recurrence in the primary and pelvic lymph nodes. Treatment according to the invention using MCO, ECO and TBH over 19 days, 5 months after diagnosis. All clinical evidence of disease disappeared. His PSA dropped to 4. Unfortunately he has developed a thrombosis in his left popliteal artery and has required hospitalisation for arterial graft. Patient is not having any urinary symptoms, however.

**CASE HISTORY 12**

JP (a female): Right mastectomy for cancer of the right breast at age of 37. Six months of cytotoxic chemotherapy after that and she was well for nine years when she developed a right supraclavicular mass treated by radiotherapy. Two years later developed a recurrence of this right lower neck mass around her brachial plexus. MRI confirmed presence of recurrence and a biopsy was positive for malignancy. Patient was given hormone therapy for 18 months without change, followed by six months of chemotherapy with slowly increasing pain and loss of sensation and increasing weakness of the right hand due to compression of the cervical plexus. Treated according to the invention with MCO and TBH over 21 day period. Complete relief of all symptoms with restoration of normal sensation and of muscle recovery of the right upper limb. MRI scan confirmed complete disappearance of the tumour in this area. There is, however, post-radiotherapy scarring in that area. The malignancy identified before microwave therapy has completely disappeared. Shortly afterwards she developed abdominal discomfort and was found to have metastases in the lower abdomen. This area had not been treated with the microwaves. Treatment was started for her but she was persuaded by her Oncologist that as one area had been cleared with the microwaves then the lower torso should also have been cleared of the cancer and she was advised to have chemotherapy. This lady survives with active disease in her abdominal cavity but not where she was treated by the method of the invention.

## CASE HISTORY 13

SC (a female aged about 58 at onset of disease): Two attacks of epilepsy and headache, confusion and inability to speak. Two week history and CT scan showed a left frontal mass probably primary malignancy. Operation showed a cystic malignancy which was later thought to be a malignant meningioma involving the brain. This appears to have arisen from the left frontal meninges. Post operatively with relief of pressure she had lost all her symptoms and treatment according to the invention was given with ECO, MCO and TBH over a 20 day period commencing 19 days after the operation. This lady was reviewed by her surgeon with a new CT scan. No clinical or radiological evidence of active malignancy is present.

## CASE HISTORY 14

ES (a male aged about 61 at onset of disease): A Grade 3 (fairly rapidly growing) transitional cell carcinoma of the bladder Stage 2 (involving the mucosa and the muscle wall of the bladder) treated by local resection and x-ray therapy over a 9 month period. The radiotherapy caused a bowel obstruction which required a bowel resection a further 9 months later. Six months later patient had obvious recurrence in the base of the bladder on the left side. Cystoscopy showed a recurrence, confirmed by CT scan and biopsy. Surgical treatment impossible in view of the radiotherapy complications. Treated according to the invention with ECO and TBH over a 20 day period commencing shortly after biopsy. Reviewed by the surgeon with cystoscopy. Cystoscopy within normal limits allowing for the distortion due to the surgery of his colonic surgery. No clinical evidence of malignancy. Biopsies of the bladder wall show minor changes with atypia but no malignancy. He remains asymptomatic.

## Claims

1. Use of a non-toxic S-substituted cysteine sulfoxide of the general formula

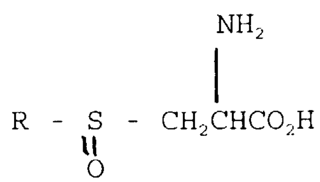

(V)

or a non-toxic salt thereof, in which R is a $C_{1-4}$ alkyl or a $C_{2-4}$ alkenyl group, for the manufacture of a composition or compositions for use as a medicament in a method of cancer therapy in a patient in which there is intravenously administered to a cancer site of the patient an effective amount of said non-toxic cysteine sulfoxide or salt thereof, and while the said non-toxic cysteine sulfoxide is present at the cancer site of the patient there is administered to the cancer site an effective dose of microwave electromagnetic radiation of frequency in the range of 425-450 MHz.

2. A use according to claim 1, wherein the non-toxic S-substituted cysteine sulfoxide is selected from the group consisting of S-methyl-L-cysteine sulfoxide, S-ethyl-L-cysteine sulfoxide, S-propyl-L-cysteine sulfoxide and S-butyl-L-cysteine sulfoxide.

3. A use according to claim 1 or 2, wherein the microwave electromagnetic radiation is administered at a frequency in the range of 432-436 MHz.

**4.** A use according to any one of the preceding claims, wherein the sulfoxide active agent is used in association with an oxidising agent.

**5.** A use according to claim 4, wherein the sulfoxide active agent is S-methyl-L-cysteine sulfoxide.

**6.** A use according to claim 4, wherein the oxidising agent is t-butyl hydroperoxide.

**7.** A use according to any one of the preceding claims, wherein the sulfoxide active agent is used in association with cystine or a non-toxic salt thereof.

**8.** A use according to any one of the preceding claims, wherein the sulfoxide active agent is used in association with oxidised glutathione or a non-toxic salt thereof.

**9.** A kit of two or more compositions containing active agents, selected from compounds of formula

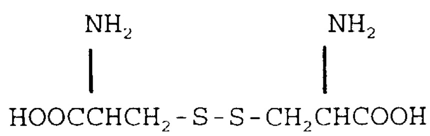

(I),

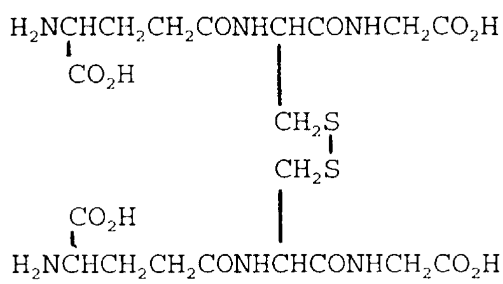

(II),

$$CH_3C(CH_3)_2OOH \quad \text{(III)},$$

and

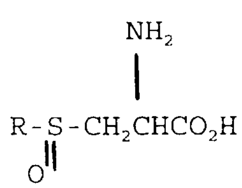

(V)

and non-toxic salts thereof, in which R is a $C_{1-4}$ alkyl or a $C_{2-4}$ alkenyl group, for rapid sequential administration in a method of cancer therapy in a patient which comprises intravenously administering to a cancer site of the patient an effective amount of the compound(s) of formula V or a non-toxic salt thereof, and while said compound of formula V is present at the cancer site administering to the cancer site an effective dose of microwave electromagnetic radiation of frequency in the range of 425-450 MHz, a first composition of the kit comprising an aqueous solution containing a first active agent selected from the agents as defined above, and a second composition of the kit comprising an aqueous solution containing a second active agent selected from the agents as defined above, with the proviso that at least one of said first and second active agents is a compound of general formula V or a salt thereof and that the composition to be administered first includes the compound of formula III.

**Patentansprüche**

1. Verwendung eines nicht toxischen S-substituierten Cystein-Sulfoxids mit der allgemeinen Formel

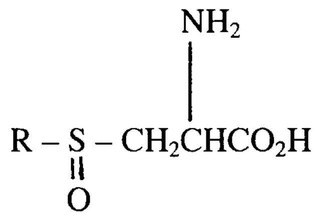

(V)

oder eines nicht toxischen Salzes desselben, bei dem R eine $C_{1-4}$-Alkyl- oder eine $C_{2-4}$-Alkenylgruppe ist, zur Herstellung einer Zusammensetzung bzw. von Zusammensetzungen zur Verwendung als Medikament bei einem Verfahren zur Krebstherapie bei einem Patienten, bei welchem eine wirksame Menge des nicht toxischen Cystein-Sulfoxids oder des Salzes desselben intravenös an den Ort der Krebserkrankung des Patienten abgegeben wird, und bei welchem, während sich das nicht toxische Cystein-Sulfoxid an dem Ort der Krebserkrankung des Patienten befindet, eine wirksame Dosis elektromagnetischer Mikrowellenstrahlung mit einer Frenquenz im Bereich von 425-450 MHz an den Ort der Krebserkrankung abgegeben wird.

2. Verwendung nach Anspruch 1, bei der das nicht toxische S-substituierte Cystein-Sulfoxid aus der Gruppe ausgewählt wird, die aus S-Methyl-L-Cystein-Sulfoxid, S-Ethyl-L-Cystein-Sulfoxid, S-Propyl-L-Cystein-Sulfoxid und S-Butyl-L-Cystein-Sulfoxid ausgewählt wird.

3. Verwendung nach Anspruch 1 oder 2, bei der die elektromagnetische Mikrowellenstrahlung mit einer Frequenz im Bereich von 432-436 MHz verabreicht wird.

4. Verwendung nach einem der vorhergehenden Ansprüche, bei der das aktive Sulfoxid-Agens zusammen mit einem Oxidationsmittel verwendet wird.

5. Verwendung nach Anspruch 4, bei der das aktive Sulfoxid-Agens S-Methyl-L-Cystein-Sulfoxid ist.

6. Verwendung nach Anspruch 4, bei der das Oxidationsmittel t-Butyl-Hydroperoxid ist.

7. Verwendung nach einem der vorhergehenden Ansprüche, bei der das aktive Sulfoxid-Agens zusammen mit Cystin oder einem nicht toxischen Salz desselben verwendet wird.

8. Verwendung nach einem der vorhergehenden Ansprüche, bei der das aktive Sulfoxid-Agens zusammen mit oxidiertem Glutathion oder einem nicht toxischen Salz desselben verwendet wird.

9. Satz aus zwei oder mehr Zusammensetzungen mit aktiven Agenzien, ausgewählt aus Verbindungen mit der Formel

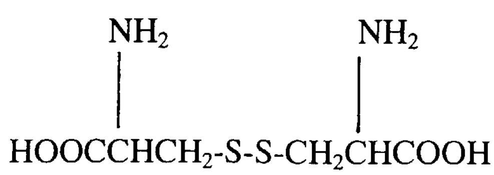

(I)

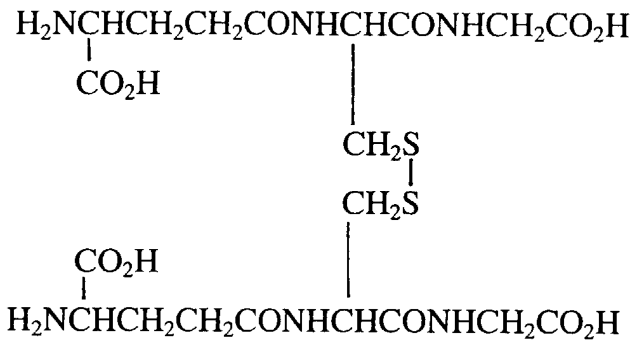 (II)

$$CH_3C(CH_3)_2OOH \quad (III)$$

und

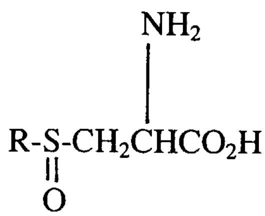 (V)

und nicht toxischen Salzen derselben, wobei R eine $C_{1-4}$-Alkyl- oder eine $C_{2-4}$-Alkenylgruppe ist, zur schnell aufeinanderfolgenden Verabreichung bei einem Verfahren zur Krebstherapie bei einem Patienten, bei welchem eine wirksame Menge der Verbindung(en) der Formel V oder ein nicht toxisches Salz derselben intravenös an einen Ort der Krebserkrankung des Patienten abgegeben wird, und bei welchem, während sich die Verbindung der Formel V an dem Ort der Krebserkrankung befindet, eine wirksame Dosis elektromagnetischer Mikrowellenstrahlung mit einer Frequenz im Bereich von 425-450 MHz an den Ort der Krebserkrankung abgegeben wird, wobei eine erste Zusammensetzung des Satzes aus einer wässrigen Lösung mit einem ersten aktiven Agens, das aus den vorstehend definierten Agenzien ausgewählt wird, besteht, und eine zweite Zusammensetzung des Satzes aus einer wässrigen Lösung mit einem zweiten aktiven Agens, das aus den vorstehend definierten Agenzien ausgewählt wird, besteht, mit der Maßgabe, dass mindestens eines der ersten und zweiten aktiven Agenzien eine Verbindung mit der allgemeinen Formel V oder ein Salz derselben ist und dass die zuerst zu verabreichende Zusammensetzung die Verbindung mit der Formel III enthält.

## Revendications

1. Utilisation d'un sulfoxyde de cystéine S-substitué non toxique de formule générale

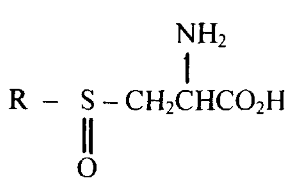 (V)

ou de l'un de ses sels non toxique, dans lequel R est un groupement alkyle en $C_{1-4}$ ou alkényle en $C_{2-4}$, afin de fabriquer une composition ou des compositions destinées à être utilisées comme un médicament dans une méthode de thérapie anticancéreuse chez un patient auquel on administre en intraveineuse une quantité efficace dudit sulfoxyde de cystéine non toxique ou de son sel en un site cancéreux du patient, et tandis que ledit sulfoxyde de cystéine non toxique est présent au niveau du site cancéreux du patient on envoie une dose efficace de rayonnement électromagnétique micro-onde à fréquence dans la gamme de 425 - 450 MHz au site cancéreux.

**2.** Utilisation selon la revendication 1, dans laquelle on choisit le sulfoxyde de cystéine S-substitué à partir du groupe consistant en sulfoxyde de S-méthyl-L-cystéine, sulfoxyde de S-éthyl-L-cystéine, sulfoxyde de S-propyl-L-cystéine et sulfoxyde de S-butyl-L-cystéine.

**3.** Utilisation selon la revendication 1 ou la revendication 2, dans laquelle on envoie le rayonnement électromagnétique micro-onde à une fréquence dans la gamme de 432 - 436 MHz.

**4.** Utilisation selon l'une des revendications précédentes, dans laquelle on utilise l'agent sulfoxyde actif en combinaison avec un agent oxydant.

**5.** Utilisation selon la revendication 4, dans laquelle l'agent sulfoxyde actif est du sulfoxyde de S-méthyl-L-cystéine.

**6.** Utilisation selon la revendication 4, dans laquelle l'agent oxydant est de l'hydroperoxyde de t-butyle.

**7.** Utilisation selon l'une des revendications précédentes, dans laquelle on utilise l'agent sulfoxyde actif en combinaison avec la cystine ou l'un des ses sels non toxique.

**8.** Utilisation selon l'une des revendications précédentes, dans laquelle on utilise l'agent sulfoxyde actif en combinaison avec le glutathion oxydé ou l'un des ses sels non toxique.

**9.** Kit de deux ou plusieurs compositions comprenant des agents actifs, choisis parmi les composés de formules

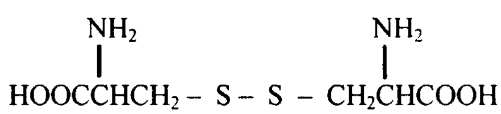

(I),

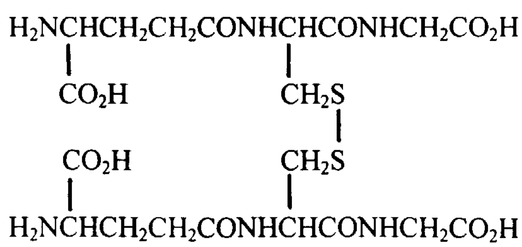

(II),

$$CH_3C(CH_3)_2OOH$$ (III),

et

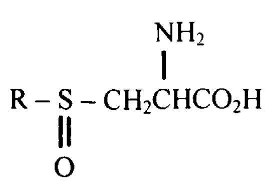

(V),

et leurs sels non toxiques, dans lesquelles R est un groupement alkyle en $C_{1-4}$ ou alkényle en $C_{2-4}$, pour une administration séquentielle rapide dans une méthode de thérapie anticancéreuse chez un patient qui consiste à administrer en intraveineuse une quantité efficace du ou des composés de formule V ou de l'un de ses sels non toxique en un site cancéreux du patient, et tandis que ledit composé de formule V est présent au niveau du site cancéreux, à envoyer une dose efficace de rayonnement électromagnétique micro-onde à une fréquence dans la gamme de 425 - 450 MHz au site cancéreux, une première composition du kit comprenant une solution aqueuse contenant un premier agent actif choisi parmi les agents définis ci-dessus, et une deuxième composition du kit comprenant une solution aqueuse contenant un deuxième agent actif choisi parmi les agents définis ci-dessus, à condition qu'au moins un parmi lesdits premiers et deuxièmes agents actifs est un composé de formule générale V ou l'un des ses sels et que la composition devant être administrée inclut tout d'abord le composé de formule III.